# EUROPEAN PATENT SPECIFICATION

(11) **EP 3 980 117 B1**
(45) Date of publication and mention of the grant of the patent: **23.04.2025**
(21) Application number: 20732528.3
(22) Date of filing: 08.06.2020
(51) Int. Cl.: A61N 5/10

(54) **MATERIAL INSERTS FOR RADIATION THERAPY**
MATERIALEINLAGEN FÜR STRAHLENTHERAPIE
INSERTS DE MATÉRIAU POUR RADIOTHÉRAPIE

(30) Priority: 10.06.2019 US 201916436671
(43) Date of publication of application: 13.04.2022
(73) Proprietor: Siemens Healthineers International AG, 6312 Steinhausen (CH); Varian Medical Systems, Inc., Palo Alto, CA 94304-1038 (US); Varian Medical Systems Particle Therapy GmbH & Co. KG, 53842 Troisdorf (DE)
(72) Inventor: PEREZ, Jessica, 6312 Steinhausen (CH); KOPONEN, Timo, 6312 Steinhausen (CH); ABEL, Eric, Palo Alto, California 94304-1038 (US); ZANKOWSKI, Corey, Palo Alto, California 94304-1038 (US); MAGLIARI, Anthony, Palo Alto, California 94304-1038 (US); SMITH, Christel, Palo Alto, California 94304-1038 (US); FOLKERTS, Michael, Palo Alto, California 94304-1038 (US); HANSEN, Bill, Palo Alto, California 94304-1038 (US); VANDERSTRAETEN, Reynald, 53842 Troisdorf (DE)
(74) Representative: Foster, Mark Charles
(86) International application number: PCT/EP2020/065841
(87) International publication number: WO 2020/249513

(56) References cited:
- WO-A1-2015/077881
- WO-A1-2015/153746
- WO-A1-2017/173443
- WO-A1-2019/018341

## Description

### BACKGROUND

The use of radiation therapy to treat cancer is well known. Typically, radiation therapy involves directing a high energy beam of radiation into a target (e.g., a tumor or lesion) in a patient.

A radiation therapy device typically includes, among other components, a platform (e.g., a table or couch) to support the patient and a nozzle that emits the radiation beam. The patient is positioned in a supine position, for example, and the nozzle directs the beam into the target (e.g., the tumor being treated).

During treatment, it is important to keep the patient as stationary (immobilized) as possible, so that the beam remains pointed at the target and at the proper place within the target. Otherwise, the radiation beam may miss parts of the target or might land on normal (healthy) tissue outside the target. Fixation or immobilization devices are used to secure a patient's position and keep the patient stationary during radiotherapy.

A standard treatment process includes scanning and imaging the patient prior to treatment to detect internal organs and accurately locate the target (e.g., the tumor). Immobilization devices customized for the patient are designed and a treatment plan is generated. The designs for the immobilization devices are sent to a manufacturer. The manufactured immobilization devices are delivered to the treatment center, where they are tested prior to beginning radiotherapy. If changes are needed, then the process of interacting with the manufacturer is repeated. The patient then returns and treatment can begin. An exemplary immobilization device for patient's head which comprises a plurality of material inserts having regular shape is described in publication WO 2015/153746 A1.

The conventional approach to providing the immobilization device described above is problematic for a variety of reasons. First, multiple patient visits are required - at least one visit is required prior to treatment in order to design the immobilization devices. Also, the need to involve a manufacturer increases costs. Furthermore, time may be lost while the immobilization devices are shipped from and perhaps back to the manufacturer.

Also during treatment, the beam nozzle and/or the patient are typically moved relative to one another so that the beam can be directed into the target from different directions/angles (beam geometries). The target may have an irregular shape and/or the amount (depth) of normal, healthy tissue on the beam path may vary depending on the beam geometry. In general, it may be necessary to shape the dose distribution delivered by a beam according to the shape and depth of the target and the beam geometry.

A range compensator is used to change (e.g., decrease) the energies of particles in a beam to affect the distance that the beam penetrate into the target. The range compensator may be located downstream of the particle accelerator before the nozzle or in the nozzle itself.

A recent radiobiology study has demonstrated the effectiveness of delivering an entire, relatively high therapeutic radiation dose to a target within a single, short period of time. This type of treatment is referred to generally herein as FLASH radiation therapy (FLASH RT). Evidence to date suggests that FLASH RT advantageously spares normal, healthy tissue from damage when that tissue is exposed to only a single irradiation for only a very short period of time. In general, because of the higher dose rates associated with FLASH RT, it is desirable to minimize the amount of time that normal, healthy tissue outside the target is irradiated. A means of achieving that is to produce a radiation treatment plan in which multiple beams do not overlap, or overlap as little as possible, outside the target. With FLASH RT, the direction/angle of the nozzle is set so that the nozzle is aimed at the target; the range compensator is adjusted to account for the beam energy, the distance to the target, and the shape of the target (the distance across the target); and then the beam is turned on and quickly turned off. The process is repeated for the next beam geometry. To reduce overall treatment time for the comfort of the patient, it is desirable to be able to quickly adjust the range compensator for the different beam geometries.

### SUMMARY

In one aspect the present invention provides an immobilization device for use in treating a patient during radiation therapy as defined in claim 1. Optional features are defined in the claims dependent thereon. In another aspect the present invention provides a computer-implemented method of radiation treatment planning as defined in claim 9. Optional features are defined in the claims dependent thereon. There is also described a computer-implemented radiation treatment method which does not form part of the invention.

Embodiments of the present invention describe systems for providing radiation therapy treatment using an immobilization device, e.g a cranial immobilization device. The immobilization device may cover a patient's head during radiation treatment and includes a material insert disposed within a shell of the immobilization device. The shell can be made of a high Z material to degrade the energy of a beam applied to the patient, and the range compensator fine tunes the depth and range of the beam so that the Bragg peak is located within a target of the patient. The range compensator is secured and supported by a scaffolding disposed in the shell so that the range compensator is located immediately before the patient.

According to one embodiment, an immobilization device for use in treating a patient during radiation therapy is disclosed. The device includes a shell, a plurality of different shaped and sized material inserts disposed in the shell, where each material insert of the plurality of material inserts respectively, and specifically shapes a distribution of a dose delivered to the patient by a respective beam of a plurality of beams emitted from a nozzle of a radiation treatment system in accordance with a treatment plan, and a scaffold component disposed in the shell operable to hold the plurality material inserts in place relative to the patient, wherein each material insert lies on a path of at least one of the plurality of beams. Each material insert has an irregular shape for shaping the respective beam to correspond to a shape or region of a target in the patient and a non-uniform thickness in accordance with a treatment plan.

According to another embodiment, a computer-implemented method of radiation treatment planning is disclosed. The method includes accessing, from a memory of a computer system, parameters for a radiation treatment plan, the parameters comprising a number of beams and beam paths relative to a position of a patient, and identifying locations on the patient for a plurality of material inserts disposed in an immobilization device (e.g. a cranial immobilization device), where each material insert of the plurality of material inserts lies on at least one of the beam paths and respectively shapes a distribution of a dose to be delivered to the patient by at least one of the beams in accordance with a treatment plan. Each material insert has an irregular shape for shaping the respective beam to correspond to a shape or region of a target in the patient and a non-uniform thickness in accordance with a treatment plan.

A computer-implemented radiation treatment method not forming part of the invention is also disclosed. The method includes accessing, from a memory of a computer system, a radiation treatment plan that prescribes a distribution of a dose to be delivered to a target in a patient by a plurality of beams emitted from a nozzle of a radiation treatment system, and controlling the nozzle to aim the plurality of beams at a plurality of material inserts positioned at different locations in an immobilization device (e.g. a cranial immobilization device), where each material insert of the plurality of material inserts is supported by a scaffold disposed in the immobilization device and respectively shapes a distribution of a respective dose delivered to the patient by a respective beam of the plurality of beams. The controlling includes aiming the nozzle at a first material insert of the plurality of material inserts and then turning on and emitting a first beam at the first material insert, and turning off the first beam and aiming the nozzle at a second material insert of the plurality of material inserts and turning on and emitting a second beam at the second material insert.

### BRIEF DESCRIPTION OF DRAWINGS

The accompanying drawings, which are incorporated in and form a part of this specification and in which like numerals depict like elements, illustrate embodiments of the present disclosure and, together with the detailed description, serve to explain the principles of the disclosure.
Figure 1 shows a block diagram of an example of a computing system upon which the embodiments described herein may be implemented.
Figure 2 is a block diagram showing selected components of a radiation treatment system upon which embodiments according to the present invention can be implemented.
Figure 3 illustrates elements of a radiation treatment system.
Figure 4 is a block diagram illustrating components in a process for creating immobilization devices in embodiments according to the present invention.
Figures 5A, 5B, 5C, and 5D illustrate immobilization devices.
Figure 6 is a flowchart of an example of computer-implemented operations for producing an immobilization device in embodiments according to the present invention.
Figure 7 is a flowchart of an example of computer-implemented operations for performing radiation treatment.
Figure 8A is a system for treating a patient during radiation therapy.
Figure 8B illustrates a perspective view of an example of a beam geometry.
Figure 8C illustrates a perspective view of an example of a beam geometry.
Figure 9 is a flowchart of an example of computer-implemented operations for radiation treatment planning in embodiments according to the present invention.
Figure 10 is a flowchart of an example of computer-implemented operations for radiation treatment.
Figure 11 is a diagram of a top-view of an exemplary immobilization device for providing cranial radiation therapy according to embodiments of the present invention.
Figure 12 is a diagram of a side-view of an exemplary immobilization device for providing cranial radiation therapy according to embodiments of the present invention.
Figure 13 depicts an exemplary cranial immobilization device for providing radiation therapy treatment using a plurality of beams depicted according to embodiments of the present invention.
Figure 14 depicts an exemplary cranial immobilization device for providing radiation therapy treatment using a variety of material inserts according to embodiments of the present invention.
Figure 15 depicts a patient-specific scaffold according to embodiments of the present invention.
Figure 16 is a flowchart of an example of computer-implemented operations for producing an immobilization device according to embodiments of the present invention.

### DETAILED DESCRIPTION

Reference will now be made in detail to the various embodiments of the present disclosure, examples of which are illustrated in the accompanying drawings. While described in conjunction with these embodiments, it will be understood that they are not intended to limit the disclosure to these embodiments. On the contrary, the disclosure is intended to cover alternatives, modifications and equivalents, which may be included within the scope of the disclosure as defined by the appended claims. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Some portions of the detailed descriptions that follow are presented in terms of procedures, logic blocks, processing, and other symbolic representations of operations on data bits within a computer memory. These descriptions and representations are the means used by those skilled in the data processing arts to most effectively convey the substance of their work to others skilled in the art. In the present application, a procedure, logic block, process, or the like, is conceived to be a self-consistent sequence of steps or instructions leading to a desired result. The steps are those utilizing physical manipulations of physical quantities. Usually, although not necessarily, these quantities take the form of electrical or magnetic signals capable of being stored, transferred, combined, compared, and otherwise manipulated in a computing system. It has proven convenient at times, principally for reasons of common usage, to refer to these signals as transactions, bits, values, elements, symbols, characters, samples, pixels, or the like.

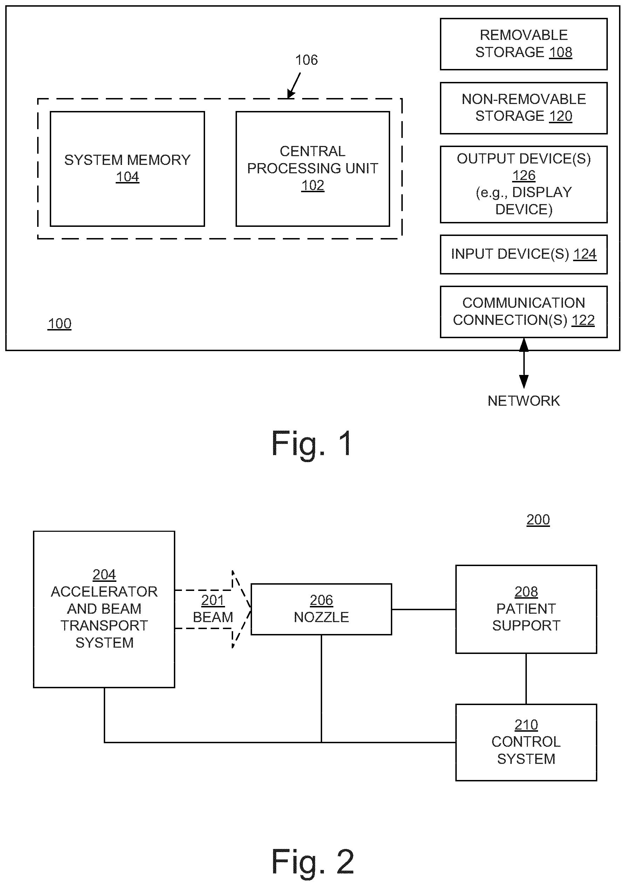

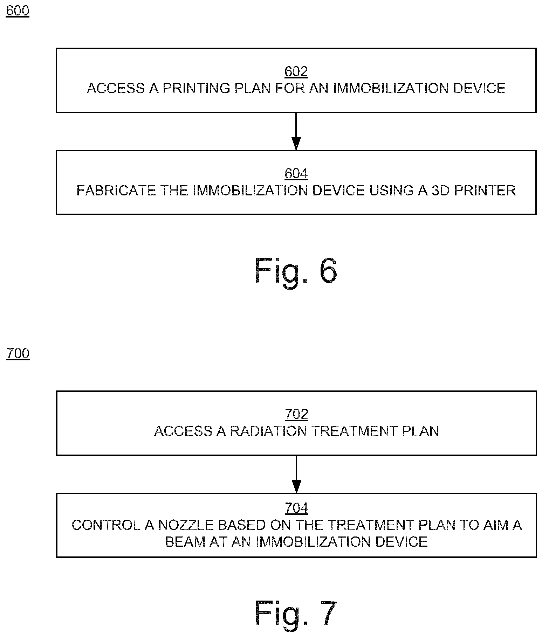

It should be borne in mind, however, that all of these and similar terms are to be associated with the appropriate physical quantities and are merely convenient labels applied to these quantities. Unless specifically stated otherwise as apparent from the following discussions, it is appreciated that throughout the present disclosure, discussions utilizing terms such as "accessing," "controlling," "identifying," "aiming," "turning on," "turning off," or the like, refer to actions and processes (e.g., the flowcharts of Figures 6, 7, 9, 10, and 16) of a computing system or similar electronic computing device or processor (e.g., the computing system 100 of Figure 1). The computing system or similar electronic computing device manipulates and transforms data represented as physical (electronic) quantities within the computing system memories, registers or other such information storage, transmission or display devices. Terms such as "dose" or "energy" generally refer to a dose or energy value; the use of such terms will be clear from the context of the surrounding discussion.

Portions of the detailed description that follows are presented and discussed in terms of a method. Although steps and sequencing thereof are disclosed in figures herein (e.g., Figures 6, 7, 9, 10, and 16) describing the operations of this method, such steps and sequencing are exemplary. Embodiments are well suited to performing various other steps or variations of the steps recited in the flowchart of the figure herein, and in a sequence other than that depicted and described herein.

Embodiments described herein may be discussed in the general context of computer-executable instructions residing on some form of computer-readable storage medium, such as program modules, executed by one or more computers or other devices. By way of example, and not limitation, computer-readable storage media may comprise non-transitory computer storage media and communication media. Generally, program modules include routines, programs, objects, components, data structures, etc., that perform particular tasks or implement particular abstract data types. The functionality of the program modules may be combined or distributed as desired in various embodiments.

Computer storage media includes volatile and nonvolatile, removable and non-removable media implemented in any method or technology for storage of information such as computer-readable instructions, data structures, program modules or other data. Computer storage media includes, but is not limited to, random access memory (RAM), read only memory (ROM), electrically erasable programmable ROM (EEPROM), flash memory or other memory technology, compact disk ROM (CD-ROM), digital versatile disks (DVDs) or other optical storage, magnetic cassettes, magnetic tape, magnetic disk storage or other magnetic storage devices, or any other medium that can be used to store the desired information and that can accessed to retrieve that information.

Communication media can embody computer-executable instructions, data structures, and program modules, and includes any information delivery media. By way of example, and not limitation, communication media includes wired media such as a wired network or direct-wired connection, and wireless media such as acoustic, radio frequency (RF), infrared and other wireless media. Combinations of any of the above can also be included within the scope of computer-readable media.

Figure 1 shows a block diagram of an example of a computing system 100 upon which the embodiments described herein may be implemented. In a basic configuration, the system 100 includes at least one processing unit 102 and memory 104. This most basic configuration is illustrated in Figure 1 by dashed line 106. The system 100 may also have additional features and/or functionality. For example, the system 100 may also include additional storage (removable and/or non-removable) including, but not limited to, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included.

In the example of Figure 1, the memory 104 includes computer-readable instructions, data structures, program modules, and the like. Depending on how it is to be used, the system 100 - by executing the appropriate instructions or the like - can be used to implement a planning system used to create immobilization devices using a three-dimensional (3D) printer, as a control system to implement a radiation treatment plan in a radiation treatment system, or to implement a system for radiation treatment planning. More generally, system 100 can be used to create immobilization devices in accordance with the present invention.

Figure 2 is a block diagram showing selected components of a radiation treatment system 200 upon which embodiments according to the present invention can be implemented. In the example of Figure 2, the system 200 includes an accelerator and beam transport system 204 that generate and/or accelerate a beam 201. Embodiments according to the invention can generate and deliver beams of various types including, for instance, proton beams, electron beams, neutron beams, photon beams, ion beams, or atomic nuclei beams (e.g., using elements such as carbon, helium, or lithium). The operations and parameters of the accelerator and beam transport system 204 are controlled so that the intensity, energy, size, and/or shape of the beam are dynamically modulated or controlled during treatment of a patient according to a radiation treatment plan.

A recent radiobiology study has demonstrated the effectiveness of delivering an entire, relatively high therapeutic radiation dose to a target within a single, short period of time. This type of treatment is referred to generally herein as FLASH radiation therapy (FLASH RT). Evidence to date suggests that FLASH RT advantageously spares normal, healthy tissue from damage when that tissue is exposed to only a single irradiation for only a very short period of time. For FLASH RT, the accelerator and beam transport system 204 can generate beams that can deliver at least four (4) grays (Gy) in less than one second, and may deliver as much as 20 Gy or 50 Gy or more in less than one second.

The nozzle 206 is used to aim the beam toward various locations (a target) within a patient supported on the patient support device 208 (e.g., a chair, couch, or table) in a treatment room. A target may be an organ, a portion of an organ (e.g., a volume or region within the organ), a tumor, diseased tissue, or a patient outline, for instance.

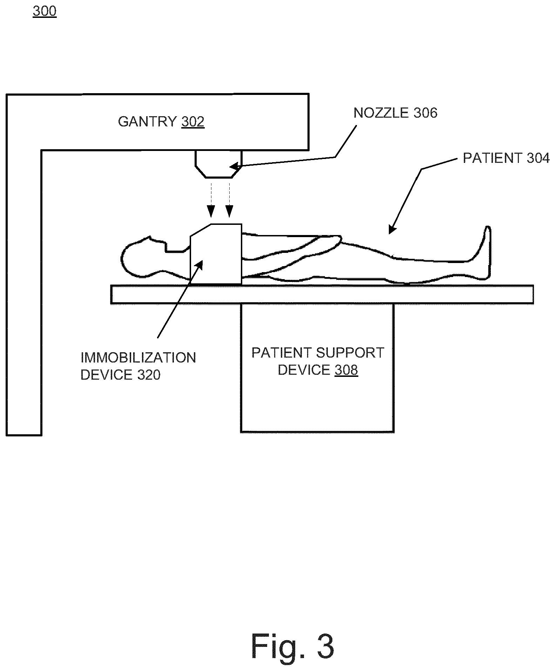

The nozzle 206 may be mounted on or may be a part of a gantry (Figure 3) that can be moved relative to the patient support device 208, which may also be moveable. In embodiments, the accelerator and beam transport system 204 are also mounted on or are a part of the gantry; in another embodiment, the accelerator and beam transport system are separate from (but in communication with) the gantry.

The control system 210 of Figure 2 receives and implements a prescribed treatment plan. In embodiments, the control system 210 includes a computing system having a processor, memory, an input device (e.g., a keyboard), and perhaps a display; the system 100 of Figure 1 is an example of such a platform for the control system 210. The control system 210 can receive data regarding the operation of the system 200. The control system 210 can control parameters of the accelerator and beam transport system 204, nozzle 206, and/or patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

### IMMOBILIZATION DEVICE INCLUDING RANGE COMPENSATOR FOR RADIATION THERAPY

Figure 3 illustrates elements of a radiation treatment system 300 for treating a patient 304. The system 300 is an example of an implementation of the radiation treatment system 200 of Figure 2, for example. In embodiments, the gantry 302 and nozzle 306 can be moved up and down the length of the patient 304 and/or around the patient, and the gantry and nozzle can move independently of one another. In embodiments, the patient support device 308 can be moved to different positions relative to the gantry 302 and nozzle 306, rotated about its longitudinal axis, rotated about a central (normal) axis, and/or tilted back and forth about a transverse axis. While the patient 304 is supine in the example of Figure 3, the invention is not so limited. For example, the patient 304 can instead be seated in a chair.

In embodiments according to the invention, an immobilization device 320 can be placed next to and against the patient 304 on the patient support device 308 during radiation therapy. The placement of the immobilization device 320 and the shape and relative size of the device shown in the example of Figure 3 are examples only. In embodiments, the immobilization device 320 is worn by the patient 304. The immobilization device 320 can be custom-designed to fit the contours of the body of the patient 304. In general, the immobilization device 320 is a patient-specific device. That is, the immobilization device 320 is designed for and used by a single patient.

The immobilization device 320 helps to establish a fixed, defined location for the patient 304 on the patient support device 308 and also helps to establish a position (e.g., posture) for the patient. An immobilization device also helps to maintain the patient in the established location and position during the course of a radiation treatment session and to re-establish and maintain the patient's location and position in subsequent treatment sessions. In embodiments according to the invention, the immobilization device 320 has a shape that provides these functionalities. Such shapes are known in the art.

Conventionally, an immobilization device is placed so that it does not obstruct the path of the beam. In contrast, in embodiments according to the invention, the immobilization device 320 is placed in the beam path, between the nozzle 306 and a target in the patient 304, so that the beam passes through the immobilization device on its way to the target.

Thus, in embodiments, another purpose of the immobilization device 320 is to ensure that any path of a radiation beam from the nozzle 306 to a target inside the patient 304 will travel through substantially the same effective thickness of matter. That is, depending on the shape of the patient's body, the location of the target in the patient, and the shape of the target, a beam may pass through different amounts (depths) of tissue if those variables are not compensated for. Similarly, two or more beams that have parallel paths may each pass through different amounts of tissue. The shape of the immobilization device 320 can be designed to compensate for these types of differences. Thus, for beams such as proton beams, electron beams, neutron beams, photon beams, ion beams, and atomic nuclei beams, a uniform (or nearly uniform) dose can be delivered across the length (depth) of the target using a beam or beams that pass through the immobilization device 320.

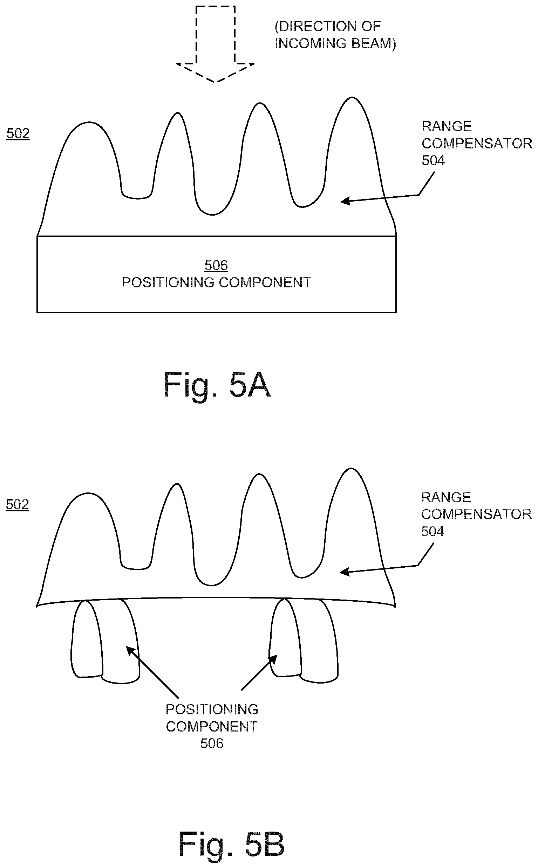

Also, for proton beams and ion beams, the immobilization device 320 can be designed to locate the Bragg peak of the beam inside the target. Specifically, the Bragg peak can be located at the distal portion or edge of the target, and then moved along the beam path toward the proximal edge of the target by changing the beam energy to achieve a Spread Out Bragg Peak (SOBP). Also, as will be described (see Figure 5A), the shape of the immobilization device 320 can be designed to achieve an SOBP.

The immobilization device 320 of Figure 3 can be advantageously utilized with FLASH RT, although the embodiments of the present invention are not so limited. In general, because of the higher dose rates associated with FLASH RT as mentioned above, it is desirable to minimize the amount of time that normal, healthy tissue outside the target is irradiated. A means of achieving that is to produce a radiation treatment plan in which beams do not overlap, or overlap as little as possible, outside the target. Another means of achieving that is to specify, during radiation treatment planning, limits for a maximum irradiation time and a minimum dose rate for normal, healthy tissue outside the target. However, it is still necessary to deliver the prescribed dose into and uniformly across the target. Immobilization devices in embodiments according to the present invention can provide a uniform dose into and across a target and thus can facilitate radiation treatment planning using FLASH RT by resolving or contributing to the resolution of that aspect of the planning.

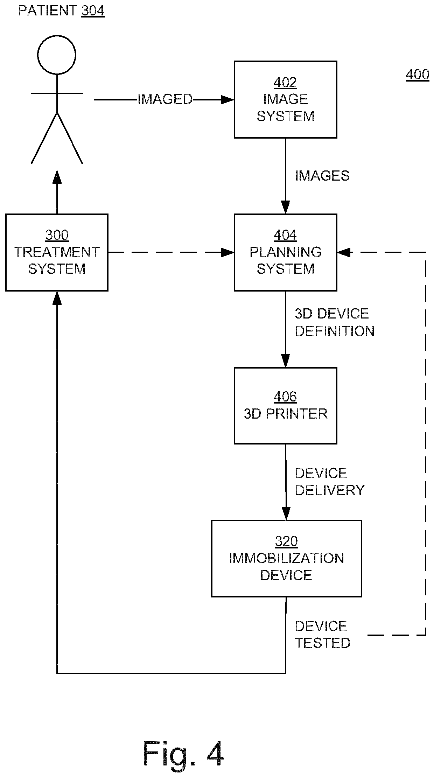

As mentioned above, immobilization devices can be created by 3D-printing using a 3D printer. Figure 4 is a block diagram illustrating components in a process 400 for creating immobilization devices in embodiments according to the present invention.

In the example of Figure 4, a patient (e.g., the patient 304) is imaged using an image system 402 that uses, for example, x-rays, magnetic resonance imaging (MRI), and/or computed tomography (CT). When CT or MRI imagery, for example, is used, a series of two-dimensional (2D) images are taken from a 3D volume. Each 2D image is an image of a cross-sectional "slice" of the 3D volume. The resulting collection of 2D cross-sectional slices can be combined to create a 3D model or reconstruction of the patient's anatomy (e.g., internal organs). The 3D model will contain organs of interest, which may be referred to as structures of interest. Those organs of interest include the organ targeted for radiation therapy (a target), as well as other organs that may be at risk of radiation exposure during treatment.

One purpose of the 3D model is the preparation of a radiation treatment plan. To develop a patient-specific radiation treatment plan, information is extracted from the 3D model to determine parameters such as organ shape, organ volume, tumor shape, tumor location in the organ, and the position or orientation of several other structures of interest as they relate to the affected organ and any tumor. The radiation treatment plan can specify, for example, how many radiation beams to use and which angle each of the beams will be delivered from.

In embodiments according to the present invention, the images from the image system 402 are input to a planning system 404. In embodiments, the planning system 404 includes a computing system having a processor, memory, an input device (e.g., a keyboard), and a display. The system 100 of Figure 1 is an example of a platform for the planning system 404.

Continuing with reference to Figure 4, the planning system 404 executes software that is capable of producing printing plans for an immobilization device or devices customized to the patient 304 and to the treatment plan devised for the patient. The software may itself translate the output of the image system 402 (e.g., the 3D model) into files that can be used by the 3D printer 406. Alternatively, software may be used by a designer to produce such files based on the output of the image system 402 and also based on the treatment plan. The printing plans may be a design for an immobilization device, or it may be a design for a mold that can be used to fabricate the immobilization device. The planning system 404 outputs the files to the 3D printer 406, which produces the immobilization device(s) and/or mold(s).

The immobilization device 320 can be produced by the 3D printer 406 using a range of different materials suitable for such a device; that is, using materials that have the necessary radiological properties. If the 3D printer 406 is not capable of using such materials, then it can instead produce a mold that can be used to produce an immobilization device made of suitable materials. The immobilization device 320 can be 3D-printed as a single piece, or it can be 3D-printed as multiple pieces that are subsequently assembled.

The immobilization device 320 so produced can be inspected and tested as part of a quality assurance plan before the device is used with a patient. If the immobilization device 320 is deficient in some aspect, the printing plans can be adjusted to correct the deficiency before the immobilization device is used.

Some or all of the process 400 can be implemented on-site (e.g., at the treatment center). Accordingly, patient-specific devices can be readily, quickly, inexpensively, effectively produced on-site without an external manufacturer, and avoiding shipping from and perhaps back to the manufacturer. The number of patient visits can be reduced because, for example, the immobilization device can be fabricated when the patient arrives for a treatment and/or because the immobilization device can be quickly modified on-site after testing for fit and/or function or while the radiation therapy is being performed. Furthermore, the immobilization devices can be recycled and do not need to be stored.

Figure 5A illustrates an immobilization device 502 that can be 3D-printed in embodiments according to the present invention. The immobilization device 502 is an example of the immobilization device 320 of Figure 3. The immobilization device 502 includes a range compensator 504 and a positioning component 506. The immobilization device 502 in general, and the range compensator 504 and positioning component 506 in particular, can be made of any suitable material or combination of materials including metal or plastic.

As discussed above, the immobilization device 502 is a patient-specific device designed or configured to hold a patient in place. The immobilization device 502 can also be designed or configured to compensate for differences in the amount of tissue that different beams may travel through, to provide a uniform dose across a target in the patient. In addition, in embodiments, the immobilization device 502 (specifically, the range compensator 504) is designed or configured to shape the distribution of the dose delivered to a patient. In embodiments, the treatment beam is a proton beam or an ion beam and the range compensator 504 is configured to locate the Bragg peak of the beam inside the target in the patient. In one such embodiment, the range compensator 504 is configured to locate the Bragg peak at the distal portion or edge of the target.

The shape of the range compensator 504 can be designed so that the Bragg peak of a proton beam or an ion beam can be moved within the target by directing the beam through different parts of the range compensator. For example, as shown in the example of Figure 5A, the range compensator 504 has a non-uniform surface facing the incoming beam. Thus, the thickness of the range compensator 504 (where thickness is measured in the direction of the beam path) is not uniform. Consequently, by aiming the beam at one part of the range compensator 504, then another, and so on, the location of the Bragg peak in the target can be moved along the beam path between the distal and proximal portions of the target to create an SOBP. That is, different thicknesses of material can be placed in the path of the beam by aiming the beam at different parts of the range compensator 504, thus affecting the energies of the particles in the beam, thereby affecting the distance the particles penetrate into the target and moving the location of the Bragg peak in the target to create an SOBP. An SOBP can also be achieved by varying the energy of the incident beam using the accelerator and beam transport system 204 (Figure 2).

Continuing with reference to Figure 5A, the positioning component 506 holds the immobilization device 502 in place relative to the patient. That is, the positioning component 506 holds the immobilization device 502 on the patient in a manner such that, if the patient moves, then the immobilization device also moves so that it is in the same location on the patient.

In embodiments, the positioning component 506 fastens the immobilization device 502 to the patient. For example, as shown in Figure 5B, the positioning component 506 may consist of or include straps that can be extended around the patient (not shown) to hold the immobilization device 502 (specifically, the range compensator 504) in place against the patient. The surface of the immobilization device 502 that faces the patient can be contoured to match the contours of the patient's body.

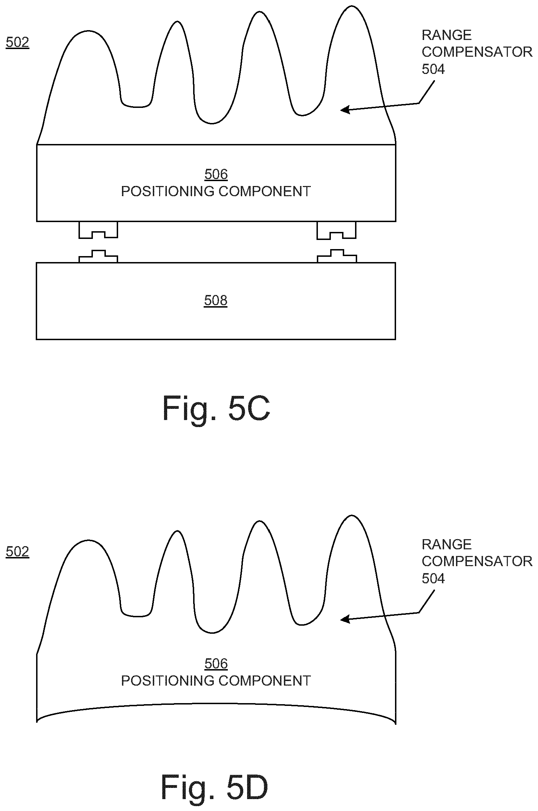

In another embodiment, with reference to Figure 5C, the positioning component 506 attaches to an item 508 worn by the patient (not shown). For example, the patient may wear a garment that includes fasteners (e.g., snaps or VELCRO^{®}) that mate with corresponding fasteners of the positioning component 506 to hold the immobilization device 502 (specifically, the range compensator 504) in place.

In embodiments, with reference to Figure 5D, the range compensator 504 and the positioning component 506 are fabricated as a single piece.

Figure 6 is a flowchart 600 of an example of computer-implemented operations for producing an immobilization device for limiting movement of a patient on a patient support device during radiation therapy in embodiments according to the present invention. The flowchart 600 can be implemented as computer-executable instructions residing on some form of computer-readable storage medium (e.g., using the computing system 100 of Figure 1).

In block 602 of Figure 6, a printing plan for an immobilization device is accessed from a memory of the computing system. The immobilization device includes features such as those described above in conjunction with Figures 3 and 5A-5D. Additional information is provided with reference to Figure 4.

In block 604 of Figure 6, a 3D printer is controlled using the printing plan to fabricate the immobilization device. Additional information is provided with reference to Figure 4.

Figure 7 is a flowchart 700 of an example of computer-implemented operations for performing radiation treatment not forming part of the present invention. The flowchart 700 can be implemented as computer-executable instructions residing on some form of computer-readable storage medium (e.g., using the computing system 100 of Figure 1).

In block 702 of Figure 7, a radiation treatment plan is accessed from a memory of the computing system. The radiation treatment plan prescribes the dose or dose distribution to be delivered to a target in a patient by an incident beam emitted from a nozzle of a radiation treatment system.

Dose threshold curves are used to specify limits for the radiation treatment plan. A dose threshold curve provides a normal (healthy) tissue sparing-dose as a function of dose rate or irradiation time. The dose threshold curves can be tissue-dependent. For instance, the dose threshold curve for the lungs may be different from that for the brain. The appropriate dose threshold curve(s) can be to establish dose limits for radiation treatment planning. For example, the appropriate (e.g., tissue-dependent) dose threshold curve can be used to determine beam directions (gantry angles).

Dose limits can include, but are not limited to: a limit on irradiation time for each sub-volume (voxel) in the target (e.g., for each voxel of target tissue, treatment time less than x1 seconds); a limit on irradiation time for each sub-volume (voxel) outside the target (e.g., for each voxel of normal tissue, treatment time less than x2 seconds; x1 and x2 may be the same or different); a limit on dose rate for each sub-volume (voxel) in the target (e.g., for each voxel of target tissue, dose rate greater than y1 Gy/sec); and a limit on dose rate for each sub-volume (voxel) outside the target (e.g., f or each voxel of normal tissue, dose rate greater than y2 Gy/sec; y1 and y2 may be the same or different). In general, the limits are intended to minimize the amount of time that normal tissue is irradiated.

In block 704, the nozzle is controlled according to the treatment plan to aim the beam at an immobilization device like that of Figures 3 and 5A-5D.

In summary, embodiments according to the present invention provide an improved immobilization device that is multi-functional. In addition to immobilizing a patient, the device can be used to shape the dose distribution within a target in the patient. In embodiments, the immobilization device includes a range compensator. In effect, in embodiments, the range compensator is moved from the nozzle of a radiation treatment system to the immobilization device. The multi-functional aspect of the immobilization device can improve radiation treatments and reduce costs. The immobilization device can be 3D-printed, which provides a number of benefits as well as explained above.

### RANGE COMPENSATORS POSITIONED ON A PATIENT FOR RADIATION THERAPY

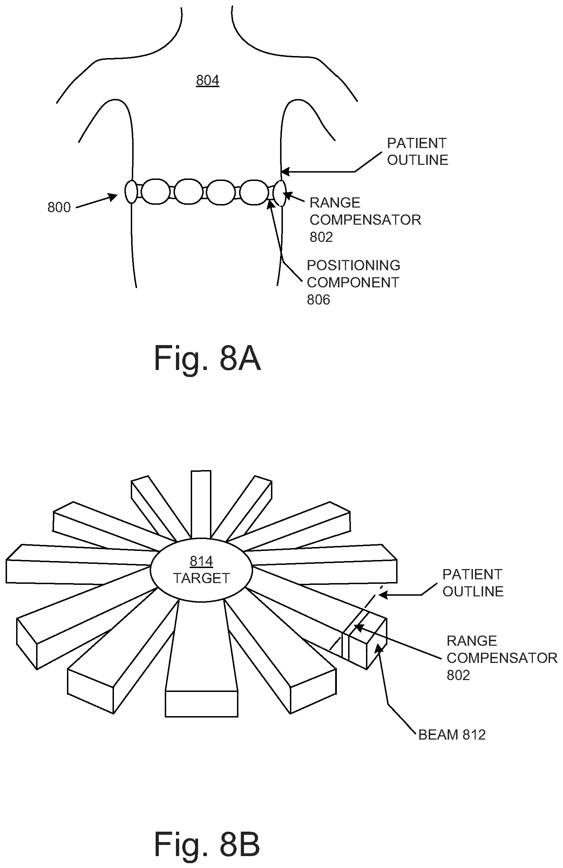

Figure 8A is a system 800 for treating a patient 804 during radiation therapy. The system 800 includes one or more range compensators (exemplified by the range compensator 802) and one or more positioning components (exemplified by the positioning component 806). In practice, each range compensator is located on the patient 804. The positioning component 806 holds the range compensator 802 in place relative to the patient 804 such that the range compensator lies on a path of a beam emitted from a nozzle of a radiation treatment system during radiation therapy.

Each of the range compensators shapes a distribution of a dose delivered to the patient 804 by the beam. The dose distribution may be relatively uniform across the target, or it may be non-uniform (e.g., the distribution may include a Bragg peak). Each range compensator can produce a different dose distribution in the target. In effect, the range compensator that conventionally is in, for example, the nozzle of a radiation treatment system is moved to locations on the patient 804. The range compensators described in conjunction with Figures 5A, 5B, 5C, and 5D are examples of the range compensator 802. One or more of the range compensators and one or more of the positioning components can be parts of an immobilization device as previously described herein. The range compensators and positioning components can be 3D-printed as previously described herein.

In embodiments, all of the range compensators are held in place on the patient 804 with a single positioning component. For example, the positioning component may be a belt worn by the patient 804, and each of the range compensators could be fastened to the belt. In another embodiment, the range compensators are held in place individually by a respective positioning component as described in conjunction with Figures 5A, 5B, 5C, and 5D.

In operation, the nozzle is aimed at a first one of the range compensators and the beam is turned on, delivering a distributed dose to the target along the beam path. That is, the path of the beam passes through the first range compensator, which affects the beam to produce a particular dose distribution in the target according to the design of the first range compensator. The first range compensator may have a non-uniform surface facing the beam as described above. In that case, the beam can be scanned across the surface of the range compensator to change the shape of the dose distribution within the target. The nozzle can be aimed at the first range compensator by moving the nozzle or by moving the patient 804 or by doing both (the patient is moved by moving the patient support device 208 of Figure 2). After the beam is turned on for the time period specified by the radiation treatment plan (see, for example, the discussion of Figure 7), the beam is turned off. The nozzle is then aimed at a second one of the range compensators (by moving the patient or the nozzle or both) and the beam is turned on again. Thus, the path of the beam now passes through the second range compensator, which affects the beam to produce a particular dose distribution in the target according to the design of the second range compensator. Like the first range compensator, the second range compensator can have a non-uniform surface facing the incoming beam and the beam can be scanned across the surface of the second range compensator. The energy or intensity of the beam transmitted through the second range compensator can be different from that transmitted through the first range compensator. The beam is turned off again after the time period specified by the radiation treatment plan. This process can be repeated for each of the range compensators. In this manner, different beam geometries are readily accommodated.

Figure 8B is a perspective view of an example of a beam geometry. In the example of Figure 8B, the beams (exemplified by beam 812) are in the same plane. The beams originate from a nozzle (not shown). Each beam can deliver a relatively high dose in a relatively short period of time. For example, each beam can deliver at least 4 Gy in less than one second, and may deliver as much as 20 Gy or 50 Gy or more in less than one second. In this example, the beams' paths overlap only within the target 814, and do not overlap outside the target in the surrounding tissue.

Figure 8B shows the range compensator 802 in the path of the beam 812. The shape of the beam 812 and the shape of the range compensator 802 shown in the figure are for illustration purposes only. In general, the range compensator 802 is located on the outside of the patient (referred to as the patient outline), either on the patient's skin or on an article of clothing or the like worn by the patient. The beam 812 is aimed so that it passes through the range compensator 802. The other beams shown in the figure can pass through other range compensators (not shown).

Although all beams are shown in Figure 8B, this does not mean that all beams are necessarily delivered at the same time or in overlapping time periods, although they can be. The number of beams delivered at any one time depends on the number of gantries or nozzles in the radiation treatment system and on the treatment plan.

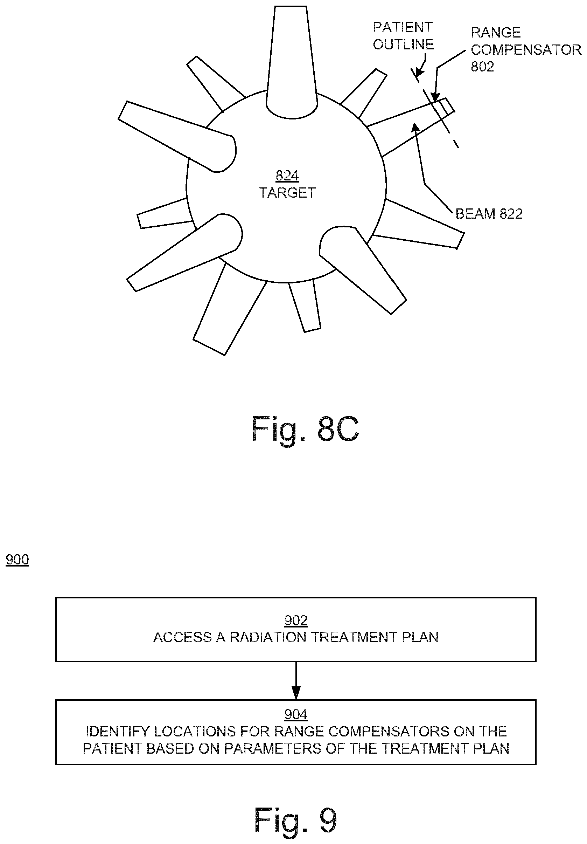

Figure 8C illustrates a perspective view of an example of a beam geometry. In the example of Figure 8C, the beams (exemplified by beam 822) are in different planes. In this example, the beams' paths overlap only within the target 824, and do not overlap outside the target in the surrounding tissue. Although all beams are shown in the figure, all beams are not necessarily delivered at the same time or in overlapping time periods as mentioned above.

Figure 8C shows the range compensator 802 in the path of the beam 822. The shape of the beam 822 and the shape of the range compensator 802 shown in the figure are for illustration purposes only. In general, the range compensator 802 is located on the outside of the patient (the patient outline) as described above. The beam 822 is aimed so that it passes through the range compensator 802. The other beams shown in the figure can pass through other range compensators (not shown).

Thus, range compensators are placed at locations on the patient 804 such that each of the beams shown in Figures 8B and 8C passes through a respective range compensator. In general, the surface of a patient can be viewed as having a number of discrete facets through which a beam may pass. From this perspective, for beams other than photon beams, each incident beam is orthogonal to a facet. A range compensator can be located on each facet.

Figure 9 is a flowchart 900 of an example of computer-implemented operations for radiation treatment planning in embodiments according to the present invention. The flowchart 900 can be implemented as computer-executable instructions residing on some form of computer-readable storage medium (e.g., using the computing system 100 of Figure 1).

In block 902 of Figure 9, parameters for a radiation treatment plan are accessed from memory of the computing system. The parameters include, for example, the number of beams and paths of the beams relative to a position of a patient on a patient support device.

In block 904, locations on the patient for range compensators are identified. Each range compensator is strategically located on the patient so that each range compensator lies on at least one of the beam paths. Each range compensator shapes a distribution of a dose to be delivered to the patient by at least one of the beams.

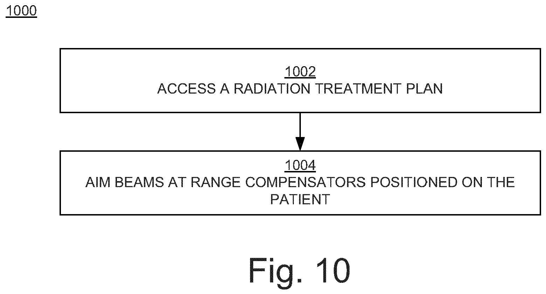

Figure 10 is a flowchart 1000 of an example of computer-implemented operations for radiation treatment not forming part of the present invention. The flowchart 1000 can be implemented as computer-executable instructions residing on some form of computer-readable storage medium (e.g., using the computing system 100 of Figure 1 to implement the control system 210 of Figure 2).

In block 1002 of Figure 10, a radiation treatment plan is accessed from memory of the computing system. The radiation treatment plan prescribes a distribution of a dose to be delivered to a target in a patient by a number of beams emitted from a nozzle of a radiation treatment system.

In block 1004, the nozzle is controlled to aim the beams at range compensators positioned at different locations on the patient. Each range compensator shapes a distribution of a dose delivered to the patient by a respective beam. The nozzle is aimed at a first range compensator, and then a first beam is turned on and emitted at the first range compensator. The first beam is then turned off, the nozzle is aimed at a second range compensator, and a second beam is turned on and emitted at the second range compensator. This process can be repeated for each of the number of beams.

### CRANIAL IMMOBILIZATION DEVICE INCLUDING RANGE COMPENSATOR FOR RADIATION THERAPY

Proton radiotherapy takes advantage of the physics of how protons deposit their energy to target tumors with high precision. As opposed to photons, which deposit their energy almost uniformly with depth, protons deposit most of the dose at the end of their path forming the so-called Bragg Peak. The depth of the Bragg Peak is proportional to the energy of the proton beam, the higher the energy, the deeper in tissue. This characteristic offers a huge dosimetric advantage in radiotherapy allowing dose deposition primarily in the tumor while preserving critical organs at risk distal to the beam. Since depth is proportional to energy, it is possible to vary the energy and determine which is required to place the Bragg Peak in the tumor.

Currently two types of accelerators are in clinical practice, cyclotrons and synchrotrons. Energy modulation is achieved directly in synchrotrons whereas cyclotrons require a separate energy selection mechanism in which material (or degrader) is placed into the proton beam path thereby reducing the beam energy to the desired range. This process also introduces lateral scatter thereby causing significant beam divergence. If the patient is far from the degrader, beam shaping elements are required to maintain beam sizes suitable for pencil beam treatment. Some beam shaping elements, such as collimators, remove particles resulting in reduced proton current thereby precluding ultra-high dose rate FLASH therapy. Therefore, ultra-high dose rate Bragg peak targeting benefits from beam degradation as close to the patient as possible where proton loss is minimized.

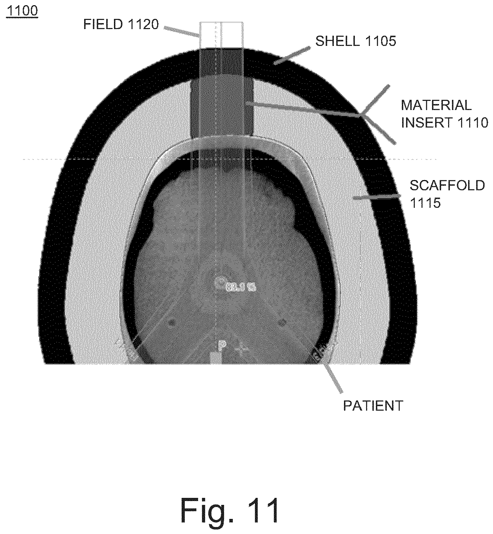

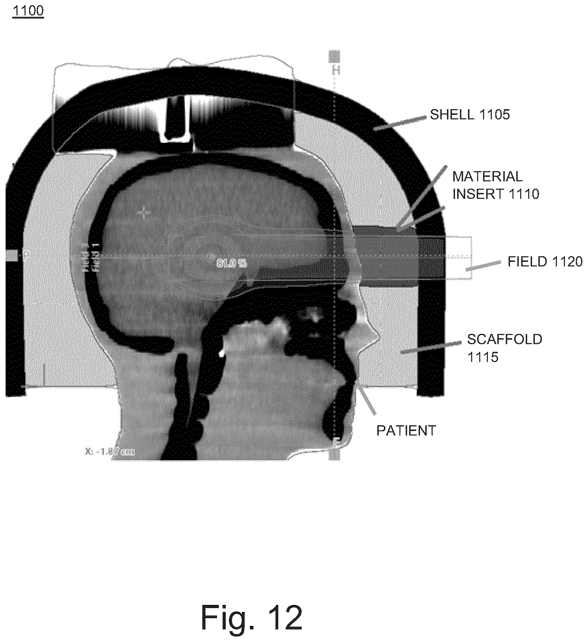

Figure 11 is a diagram of a top-view of an exemplary immobilization device 1100 for providing cranial radiation therapy including layers of materials for degrading the energy of a field or beam depicted according to embodiments of the present invention. Removable material insert 1110 can be a range compensator, range modulator, or a collimator to provide patient and beam specific depth and range modulation to shape the dose delivered to a specific target. As describe below, the scaffold 115 accommodates material inserts. Figure 12 shows a side-view of the exemplary cranial immobilization device 1100. The shell 1105 and material insert 1110 of the immobilization device 1100, as shown in Figures 11 and 12, can provide a uniform dose into and across a target and thus can facilitate radiation treatment planning using FLASH RT by resolving or contributing to the resolution of that aspect of the planning. Although, in the embodiment, a cranial immobilization device 1100 for use in cranial radiation therapy is described, it should be understood that the invention is applicable to immobilization devices for various patient body parts other than the cranium or head - such as parts of the patient torso - for use in other types of radiation therapy.

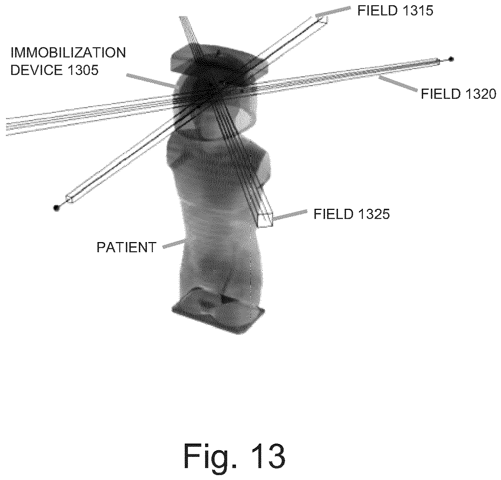

The specific number, shape, size, and type of material of the material inserts (e.g., material insert 1110) can be determined according to a treatment plan, and the treatment plan can be generated using an image system as described above with respect of Figure 4, for example. Although the immobilization device 1100 depicted in Figures 11 and 12 is illustrated using a single material insert 1110, embodiments of the cranial immobilization device described herein can accommodate up to 15 material inserts simultaneously (see Figure 13). By using a number of different material inserts disposed in the immobilization device 1100, the target can be treated using a number of non-coplanar gantry angles and the Bragg peak can be located at different depths according to the gantry angle and the location of the target. For example, different materials with different densities can be utilized to control the depth of the Bragg peak according to a treatment plan.

A patient-specific scaffold or support material 1115 is disposed between the outer layer or "shell" 1105 and the head of the patient. The shell has a helmet shape and encloses the scaffold 1115 and the material inserts 1110. To improve the comfort of the patient while wearing the cranial immobilization device 1100, the scaffold 1115 includes openings located near the patient's nose to facilitate breathing during treatment while the cranial immobilization device is worn by the patient, and includes holes near the patient's eyes so that the patient's eyes are not contacted or covered by the scaffold 1115. Moreover, the scaffold 1115 includes holes for accommodating and supporting the material inserts (e.g., material insert 1110) used to provide patient and beam specific depth and range modulation according to the treatment plan.

The cranial immobilization device 1100 in general, including the shell 1105 and the material insert 1110, can be made of any suitable material or combination of materials including metal or plastic. For example, the shell 1105 can be made of a hard plastic, and the material insert 1110 can be made of high atomic number (Z) materials, such as brass or boron. The shell 1105 provides an initial layer of material for degrading the energy of field 1120 applied to the patient for range compensation, and removable material insert 1110 fine tunes the energy of field 1120 to provide patient and beam specific depth and range modulation to shape the dose delivered to the target. Moreover, the shell 1105 and/or the scaffold 1115 advantageously limit the movement of the patent (e.g., the patient's head) during treatment. The shell 1105 and/or the scaffold 1115 may help to establish a fixed, defined location for the patient (or part of the patient, such as the head) on a patient support device (e.g. a patient support device like that shown at 308 in Figure 3) and also helps to establish a position (e.g., posture) for the patient. An immobilization device also may help to maintain the patient in the established location and position during the course of a radiation treatment session and to re-establish and maintain the patient's location and position in subsequent treatment sessions. In embodiments according to the invention, the shell 1105 and/or the scaffold 1115 may have a shape that provides these functionalities.

The scaffold 1115 is made of a rigid material for supporting relatively heavy material inserts and can be made, for example, by a 3D printer or by any other suitable means. For example, the scaffold 1115 can be made of a 3D printed mesh that can support and secure heavy material inserts made of brass or boron.

The material insert 1110 can be made from different materials depending on the density required by the treatment plan. For example, the material inserts can be made of plastic, ridge filters, or a 3D printed material (filament), or acrylic. The material inserts can also be formed by pouring a fluid material into a support hole or mold and allowing the fluid material to solidify in the shape of the mold. In some cases, the density of air may be sufficient to fine tune the beam energy, and therefore a hole can be provided in the scaffold 1115 without a corresponding insert, in some cases. The material insert 1110 can be enclosed or surrounded with a high Z material (e.g., an aperture) to provide additional range modulation. Moreover, the material insert 1110 can include a collimator to sharpen the lateral penumbra of the beam.

The scaffold 1115 and the material insert 1110 in accordance with embodiments of the present invention are customized for the patient according to the treatment plan. In general, the immobilization device 1100 is customized according to a 3D model of a patient's head and accounts for the location and shape of the target, the shape and dimensions of the patient's head, and the distance each beam applied to target must travel to reach the target. The shape of the material insert 1110 is an irregular/complex shape for shaping the beam to correspond to a shape or region of the target (e.g., an edge), and the material insert 1110 has a non-uniform thickness according to the treatment plan. The scaffold 1115 is fabricated or modified accordingly with holes for accommodating the shape of the material insert 1110. The scaffold 1115 and the material insert 1110 are fabricated for the patient according to the treatment plan. The shell 1105 can be a modified as needed, or can be constructed generically in different sizes, such as a "large" size and a "small" size that will accommodate most patients.

Figure 13 depicts an exemplary cranial immobilization device 1305 for providing radiation therapy treatment using a plurality of beams depicted according to embodiments of the present invention. Beam fields 1315, 1320, and 1325 are applied to a target located within the head of the patient (e.g., a brain tumor), and each beam field corresponds to a material insert (e.g., a range compensator, range modulator, or a collimator) disposed on/in the immobilization device 1305. Each material insert (not pictured) can be made of different materials having a broad range of densities. Advantageously, the radiation therapy treatment can apply the beam fields 1315, 1320, and 1325 without requiring machine changes between treatments. And because the positioning of the patient and the immobilization device 1305, including the material inserts, can be performed pre-treatment, therefore the patient does not need to be repositioned during treatment, thereby increasing the efficiency of the treatment compared to existing techniques that re-position the patient multiple times during treatment. Moreover, by performing energy degradation immediately at the patient surface using the material inserts for each beam field 1315, 1320, and 1325, beam spreading is significantly reduced compared to existing treatment systems that degrade the beam energy immediately after the nozzle emitting the beam.

The immobilization device 1305 of Figure 13 can be advantageously utilized with FLASH RT, although embodiments of the present invention are not so limited. In general, because of the higher dose rates associated with FLASH RT as mentioned above, it is desirable to minimize the amount of time that normal, healthy tissue outside the target is irradiated. A means of achieving that is to produce a radiation treatment plan in which beams do not overlap, or overlap as little as possible, outside the target. Another means of achieving that is to specify, during radiation treatment planning, limits for a maximum irradiation time and a minimum dose rate for normal, healthy tissue outside the target. However, it is still necessary to deliver the prescribed dose into and uniformly across the target. Therefore, the shell and material inserts of the immobilization device 1305 can perform energy modulation/compensation to provide a uniform dose into and across a target and thus can facilitate radiation treatment planning using FLASH RT by resolving or contributing to the resolution of that aspect of the planning. In this way, very high energy (e.g., 250 MeV) can be applied to the target and the immobilization device performs range modulation to stop the Bragg peak at the desired position.

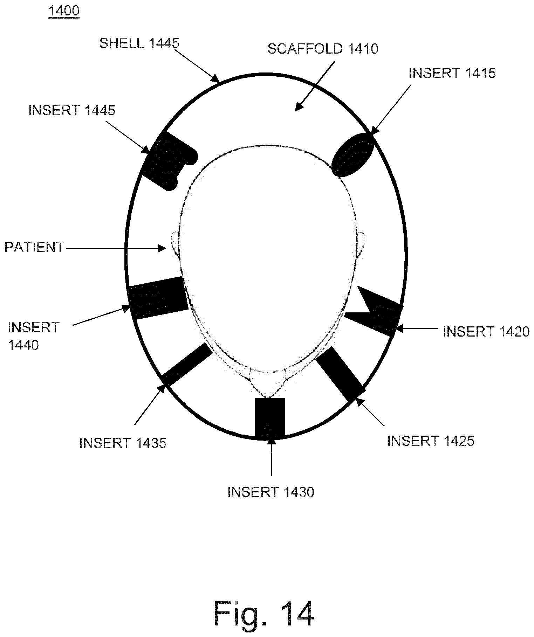

Figure 14 depicts an exemplary cranial immobilization device 1400 for providing radiation therapy treatment to a patient using a variety of material inserts 1415, 1420, 1425, 1430, 1435, and 1440 according to embodiments of the present invention. Each material insert has a specific size and shape and is made from different materials with different densities according to a treatment plan. The material inserts have an irregular shape to produce a beam shape corresponding to a region or edge of a target. When a beam is applied to the various material inserts at a specific gantry angle, the material inserts perform range compensation/modulation based on the size, shape, and density of the respective material insert. The scaffold 1410 is customized to accommodate the material inserts so that the inserts are supported securely between the patient and the shell 1450. For example, the scaffold can be manufactured by a 3D printer to include support holes corresponding to the shape of each material insert, or a mesh can be manufactured and support holes corresponding to each material inserts are cut out or drilled out of the scaffold 1410.

Each material insert is irradiated by a beam at a corresponding beam angle defined in the treatment plan. The beams can deliver a relatively high dose in a relatively short period of time. For example, each beam can deliver at least 4 Gy in less than one second, and may deliver as much as 20 Gy or 50 Gy or more in less than one second.

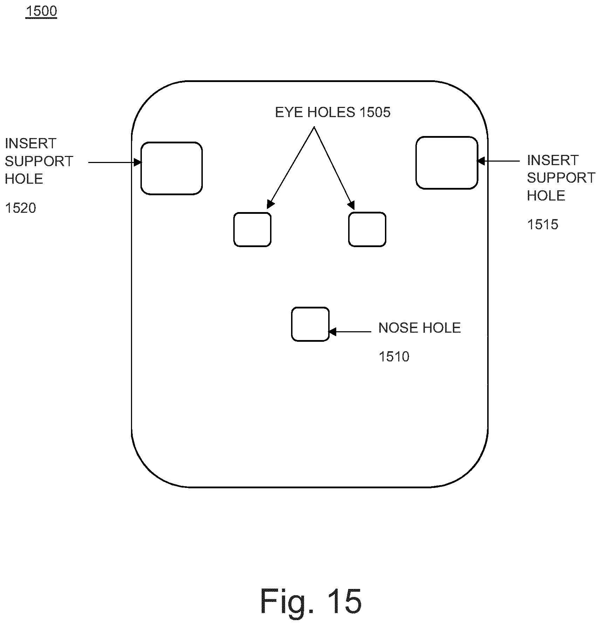

Figure 15 depicts a patient-specific scaffold 1500 according to embodiments of the present invention. The scaffold 1500 is disposed between a hollow helmet shaped shell and the head of the patient and includes holes 1505 located in front of the patient's eyes and holes 1510 located near the patient's nose. The holes 1505 and 1510 improve the comfort of the patient while wearing a cranial immobilization device. Moreover, the scaffold 1500 includes support holes 1515 and 1520 for accommodating material inserts used to provide patient and beam specific depth and range modulation according to the treatment plan. The scaffold 1500 is made of a rigid material for supporting relatively heavy material inserts and can be made by a 3D printer. For example, the scaffold 1500 can be made of a 3D printed mesh that can support and secure heavy material inserts made of brass or boron.

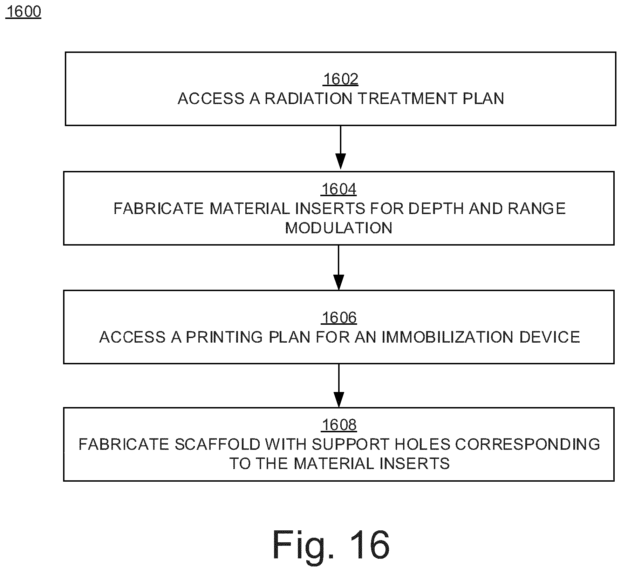

Figure 16 is a flowchart 1600 of an example of computer-implemented operations for producing an immobilization device (e.g., a cranial immobilization device) according to embodiments of the present invention. The flowchart 1600 can be implemented as computer-executable instructions residing on some form of computer-readable storage medium (e.g., using the computing system 100 of Figure 1).

In block 1602 of Figure 16, parameters for a radiation treatment plan are accessed from memory of the computing system. The parameters include, for example, the number of beams and paths of the beams relative to a position of a patient.

In block 1604 of Figure 16, material inserts are fabricated that provide depth and range modulation for the immobilization device according to the treatment plan. The material inserts are supported by the scaffold fabricated in step 1608 and can have a non-uniform shape in order to provide the desired range modulation and depth modulation of the treatment plan. Moreover, the material inserts can be made from high Z materials, such as brass and boron. The size, shape, and density of the material insert can be determined according to the radiation treatment plan which can include 3D imaging. The material insert can be a range compensator, range modulator, or a collimator. According to some embodiments, the material inserts are printed using a 3D printer according to a printing plan (e.g., the printing plan accessed in block 1606).

In block 1606 of Figure 16, a printing plan for an immobilization device (e.g., a cranial immobilization device) is accessed from a memory of the computing system. The immobilization device includes features such as those described above in conjunction with Figures 11-14. Additional information is provided with reference to Figures 11 and 12.

In block 1608 of Figure 16, a 3D printer is controlled using the printing plan to fabricate a scaffold to support and secure the material inserts. The scaffold can be made from a 3D printed mesh and can be fabricated with holes corresponding to the material inserts to support and secure the material inserts at a location close to the patient. In this way, the beam is degraded immediately at the patient surface to advantageously reduce lateral beam spreading.

In summary, range compensators in embodiments according to the invention can be used to shape dose distribution in the target in lieu of, but also in combination with, a conventional range compensator. By strategically locating different sized and shaped range compensators or material inserts on the patient, different beam geometries are readily accommodated. For radiation therapy including FLASH RT, it is not necessary to wait until a range compensator is adjusted when the beam geometry changes; instead, a properly configured range compensator is already in place in accordance with the treatment plan. Thus, radiation therapy including FLASH RT can be quickly performed, thereby facilitating patient comfort. Range compensators or material inserts as inserts into the scaffold in embodiments according to the present invention also can be used to provide the prescribed dose inside the target and thus can facilitate radiation treatment planning using FLASH RT by making it easier to address that aspect of the planning.

The invention is defined by the appended claims.

## Claims

1. An immobilization device for securing a patient's position for use in treating the patient during radiation therapy, the device comprising:
a shell component (1105);
a plurality of material inserts (1110) disposed in the shell component (1105), wherein each material insert of the plurality of material inserts (1110) is disposed in the shell component (1105) to lie on a path of at least one of a plurality of beams emitted from a nozzle of a radiation treatment system and is configured to respectively shape a distribution of a dose delivered to the patient by a respective beam of the plurality of beams; and
a scaffold (1115) component disposed in the shell component and operable to hold the plurality material inserts in place relative to the patient;
the immobilization device being **characterized by** each material insert having an irregular shape for shaping the respective beam to correspond to a shape or region of a target in the patient and a non-uniform thickness in accordance with a treatment plan.

2. The device of Claim 1, wherein each of the plurality of material inserts comprise at least one of: a range compensator; a range modulator; and a collimator.

3. The device of Claim 1 or 2, wherein the plurality of beams comprises beams selected from the group consisting of: proton beams; and ion beams, and wherein a respective beam of the plurality of beams passes through a respective material insert to locate a Bragg peak of the respective beam at a predetermined location inside a target of the patient in accordance with the treatment plan.

4. The device of Claim 1, 2 or 3, wherein the shell component and scaffold limit movement of the patient, and wherein the shell is in the shape of a helmet to accommodate the patient's head.

5. The device of any one of claims 1 to 4, wherein the shell component comprises a high Z material and is operable to perform range compensation for the plurality of beams.

6. The device of any one of claims 1 to 5, wherein the scaffold comprises a plurality of holes, each hole having a shape corresponding to a respective material insert of the plurality of material inserts to secure a respective material insert near a surface of the patient.

7. The device of any one of claims 1 to 6, wherein the scaffold is fabricated using 3D printing.

8. The device of any one of claims 1 to 7, wherein the plurality of material inserts comprise ridge filters.

9. A computer-implemented method of radiation treatment planning, the method comprising:
accessing, from a memory of a computer system, parameters for a radiation treatment plan, the parameters comprising a planned number of beams and planned beam paths relative to a position of a patient; and
identifying locations on the patient for a plurality of material inserts disposed in a patient body part immobilization device, wherein each material insert of the plurality of material inserts is disposed in the patient body part immobilization device to lie on at least one of the planned beam paths and is arranged and/or configured to respectively shape a distribution of a dose to be delivered to the patient by at least one of the planned beams emitted from a nozzle of a radiation treatment system;
wherein each material insert has an irregular shape for shaping the respective beam to correspond to a shape or region of a target in the patient and a non-uniform thickness in accordance with the treatment plan.

10. The method of Claim 9 , wherein each of the plurality of material inserts comprise at least one of: a range compensator; a range modulator; and a collimator.

11. The method of Claim 9 or 10, wherein the beams comprise beams selected from the group consisting of: proton beams; and ion beams, and wherein each material insert of the plurality of material inserts is configured to locate a Bragg peak of the at least one beam inside a target in the patient at a predetermined position in accordance with the treatment plan.

12. The method of Claim 9 , 10 or 11, wherein the plurality of material inserts are supported by a scaffold disposed in the cranial immobilization device.

13. The method of Claim 12, further comprising:
accessing, from the memory of the computer system, a printing plan for the immobilization device; and
controlling a three-dimensional printer using the printing plan to fabricate the scaffold, wherein the scaffold comprises holes for accommodating the plurality of material inserts.

14. The method of any one of claims 9 to 13, wherein the immobilization device is operable to cover a head and limits movement of the patient relative to the beams.

15. The method of any one of claims 9 to 14, wherein the immobilization device is as claimed in any one of claims 1 to 8.

## Patentansprüche

1. Immobilisierungsvorrichtung zum Sichern einer Patientenposition zur Verwendung bei der Behandlung des Patienten während einer Strahlentherapie, wobei die Vorrichtung Folgendes umfasst:
eine Schalenkomponente (1105);
eine Vielzahl von Materialeinlagen (1110), die in der Schalenkomponente (1105) angeordnet ist, wobei jede Materialeinlage der Vielzahl von Materialeinlagen (1110) so in der Schalenkomponente (1105) angeordnet ist, dass sie auf einem Weg von mindestens einem von einer Vielzahl von Strahlen liegt, die von einer Düse eines Strahlenbehandlungssystems emittiert werden, und dazu konfiguriert ist, jeweils eine Verteilung einer Dosis zu formen, die durch einen jeweiligen Strahl der Vielzahl von Strahlen an den Patienten abgegeben wird; und
eine Gerüstkomponente (1115), die in der Schalenkomponente angeordnet und funktionsfähig ist, die Vielzahl von Materialeinlagen in Bezug auf den Patienten an Ort und Stelle zu halten;
wobei die Immobilisierungsvorrichtung **dadurch gekennzeichnet ist, dass** jede Materialeinlage eine unregelmäßige Form zum Formen des jeweiligen Strahls aufweist, um einer Form oder einem Bereich eines Ziels in dem Patienten zu entsprechen, und eine ungleichmäßige Dicke gemäß einem Behandlungsplan aufweist.

2. Vorrichtung nach Anspruch 1, wobei jede der Vielzahl von Materialeinlagen mindestens einen von einem Bereichskompensator, einem Bereichsmodulator und einem Kollimator umfasst.

3. Vorrichtung nach Anspruch 1 oder 2, wobei die Vielzahl von Strahlen Strahlen umfasst, die aus der Gruppe bestehend aus Protonenstrahlen und Ionenstrahlen ausgewählt sind und wobei ein jeweiliger Strahl der Vielzahl von Strahlen eine jeweilige Materialeinlage durchquert, um einen Bragg-Peak des jeweiligen Strahls an einer vorbestimmten Stelle innerhalb eines Ziels des Patienten gemäß dem Behandlungsplan zu lokalisieren.

4. Vorrichtung nach Anspruch 1, 2 oder 3, wobei die Schalenkomponente und das Gerüst die Bewegung des Patienten begrenzen und wobei die Schale in Form eines Helms ausgebildet ist, um den Kopf des Patienten aufzunehmen.

5. Vorrichtung nach einem der Ansprüche 1 bis 4, wobei die Schalenkomponente ein High-Z-Material umfasst und funktionsfähig ist, eine Bereichskompensation für die Vielzahl von Strahlen durchzuführen.

6. Vorrichtung nach einem der Ansprüche 1 bis 5, wobei das Gerüst eine Vielzahl von Löchern umfasst, wobei jedes Loch eine Form aufweist, die einer jeweiligen Materialeinlage der Vielzahl von Materialeinlagen entspricht, um eine jeweilige Materialeinlage nahe einer Oberfläche des Patienten zu sichern.

7. Vorrichtung nach einem der Ansprüche 1 bis 6, wobei das Gerüst unter Verwendung von 3D-Druck hergestellt ist.

8. Vorrichtung nach einem der Ansprüche 1 bis 7, wobei die Vielzahl von Materialeinlagen Ridge-Filter umfasst.

9. Computerimplementiertes Verfahren zur Strahlenbehandlungsplanung, umfassend:
Zugreifen auf Parameter für einen Strahlenbehandlungsplan aus einem Speicher eines Computersystems, wobei die Parameter eine geplante Anzahl von Strahlen und geplante Strahlenwege relativ zu einer Position eines Patienten umfassen; und
Identifizieren von Stellen an dem Patienten für eine Vielzahl von Materialeinlagen, die in einer Patientenkörperteil-Immobilisierungsvorrichtung angeordnet sind, wobei jede Materialeinlage der Vielzahl von Materialeinlagen so in der Patientenkörperteil-Immobilisierungsvorrichtung angeordnet ist, dass sie auf mindestens einem der geplanten Strahlenwege liegt, und so angeordnet und/oder konfiguriert ist, dass sie jeweils eine Verteilung einer Dosis formt, die durch mindestens einen der geplanten Strahlen, die von einer Düse eines Strahlenbehandlungssystems emittiert werden, an den Patienten abgegeben wird;
wobei jede Materialeinlage eine unregelmäßige Form zum Formen des jeweiligen Strahls aufweist, um einer Form oder einem Bereich eines Ziels in dem Patienten zu entsprechen, und eine ungleichmäßige Dicke gemäß einem Behandlungsplan aufweist.

10. Verfahren nach Anspruch 9, wobei jede der Vielzahl von Materialeinlagen mindestens einen von einem Bereichskompensator, einem Bereichsmodulator und einem Kollimator umfasst.

11. Verfahren nach Anspruch 9 oder 10, wobei die Strahlen Strahlen umfassen, die aus der Gruppe bestehend aus Protonenstrahlen und Ionenstrahlen ausgewählt sind, und wobei jede Materialeinlage der Vielzahl von Materialeinlagen dazu konfiguriert ist, einen Bragg-Peak des mindestens einen Strahls innerhalb eines Ziels in dem Patienten an einer vorbestimmten Position gemäß dem Behandlungsplan zu lokalisieren.

12. Verfahren nach Anspruch 9, 10 oder 11, wobei die Vielzahl von Materialeinlagen von einem Gerüst getragen wird, das in der cranialen Immobilisierungsvorrichtung angeordnet ist.

13. Verfahren nach Anspruch 12, ferner umfassend:
Zugreifen auf einen Druckplan für die Immobilisierungsvorrichtung aus dem Speicher des Computersystems; und
Steuern eines dreidimensionalen Druckers unter Verwendung des Druckplans zum Herstellen des Gerüsts, wobei das Gerüst Löcher zum Aufnehmen der Vielzahl von Materialeinlagen aufweist.

14. Verfahren nach einem der Ansprüche 9 bis 13, wobei die Immobilisierungsvorrichtung funktionsfähig ist, einen Kopf zu bedecken und die Bewegung des Patienten relativ zu den Strahlen zu begrenzen.

15. Verfahren nach einem der Ansprüche 9 bis 14, wobei die Immobilisierungsvorrichtung einem der Ansprüche 1 bis 8 entspricht.

## Revendications

1. Dispositif d'immobilisation pour sécuriser la position d'un patient en vue du traitement du patient pendant une radiothérapie, le dispositif comprenant :
un composant de coque (1105) ;
une pluralité d'inserts de matériau (1110) disposés dans le composant de coque (1105), dans lequel chaque insert de matériau de la pluralité d'inserts de matériau (1110) est disposé dans le composant de coque (1105) pour se trouver sur un trajet d'au moins l'un d'une pluralité de faisceaux émis à partir d'une buse d'un système de traitement par rayonnement et est configuré pour façonner respectivement une distribution d'une dose délivrée au patient par un faisceau respectif de la pluralité de faisceaux ; et
un composant d'échafaudage (1115) disposé dans le composant de coque et pouvant être utilisé pour maintenir la pluralité d'inserts de matériau en place par rapport au patient ;
le dispositif d'immobilisation étant **caractérisé en ce que** chaque insert de matériau a une forme irrégulière pour façonner le faisceau respectif afin de correspondre à une forme ou à une région d'une cible chez le patient et une épaisseur non uniforme conformément à un plan de traitement.

2. Dispositif selon la revendication 1, dans lequel chacun de la pluralité d'inserts de matériau comprend au moins l'un des éléments suivants : un compensateur de plage ; un modulateur de plage ; et un collimateur.

3. Dispositif selon la revendication 1 ou 2, dans lequel la pluralité de faisceaux comprend des faisceaux choisis dans un groupe composé de : faisceaux de protons ; et faisceaux d'ions, et dans lequel un faisceau respectif de la pluralité de faisceaux traverse un insert de matériau respectif pour localiser un pic de Bragg du faisceau respectif à un emplacement prédéterminé à l'intérieur d'une cible du patient conformément au plan de traitement.

4. Dispositif selon la revendication 1, 2 ou 3, dans lequel le composant de coque et l'échafaudage limitent le mouvement du patient, et dans lequel la coque a la forme d'un casque pour loger la tête du patient.

5. Dispositif selon l'une quelconque des revendications 1 à 4, dans lequel le composant de coque comprend un matériau à Z élevé et peut être utilisé pour exécuter une compensation de portée pour la pluralité de faisceaux.

6. Dispositif selon l'une quelconque des revendications 1 à 5, dans lequel l'échafaudage comprend une pluralité de trous, chaque trou ayant une forme correspondant à un insert de matériau respectif de la pluralité d'inserts de matériau pour fixer un insert de matériau respectif à proximité d'une surface du patient.

7. Dispositif selon l'une quelconque des revendications 1 à 6, dans lequel l'échafaudage est fabriqué à l'aide d'une impression 3D.

8. Dispositif selon l'une quelconque des revendications 1 à 7, dans lequel la pluralité d'inserts de matériau comprennent des filtres de crête.

9. Procédé de planification de traitement par rayonnement mis en œuvre par ordinateur comprenant :
l'accès, à partir d'une mémoire d'un système informatique, à des paramètres pour un plan de traitement par rayonnement, les paramètres comprenant un nombre prévu de faisceaux et des trajets de faisceau prévus par rapport à une position d'un patient ; et
l'identification d'emplacements sur le patient pour une pluralité d'inserts de matériau disposés dans un dispositif d'immobilisation de partie du corps du patient, dans lequel chaque insert de matériau de la pluralité d'inserts de matériau est disposé dans le dispositif d'immobilisation de partie du corps du patient pour se trouver sur au moins l'un des trajets de faisceau prévus et est agencé et/ou configuré pour façonner respectivement une distribution d'une dose à délivrer au patient par au moins l'un des faisceaux prévus émis à partir d'une buse d'un système de traitement par rayonnement ;
dans lequel chaque insert de matériau a une forme irrégulière pour façonner le faisceau respectif afin de correspondre à une forme ou à une région d'une cible chez le patient et une épaisseur non uniforme conformément au plan de traitement.

10. Procédé selon la revendication 9, dans lequel chacun de la pluralité d'inserts de matériau comprend au moins l'un des éléments suivants : un compensateur de plage ; un modulateur de plage ; et un collimateur.

11. Procédé selon la revendication 9 ou 10, dans lequel les faisceaux comprennent des faisceaux choisis dans un groupe composé de : faisceaux de protons ; et faisceaux d'ions, et dans lequel chaque insert de matériau de la pluralité d'inserts de matériau est configuré pour localiser un pic de Bragg de l'au moins un faisceau à l'intérieur d'une cible chez le patient à une position prédéterminée conformément au plan de traitement.

12. Procédé selon la revendication 9, 10 ou 11, dans lequel la pluralité d'inserts de matériau sont supportés par un échafaudage disposé dans le dispositif d'immobilisation crânienne.

13. Procédé selon la revendication 12, comprenant également :
l'accès, à partir de la mémoire du système informatique, à un plan d'impression du dispositif d'immobilisation ; et
la commande d'une imprimante tridimensionnelle à l'aide du plan d'impression pour fabriquer l'échafaudage, dans lequel l'échafaudage comprend des trous destinés à loger la pluralité d'inserts de matériau.

14. Procédé selon l'une quelconque des revendications 9 à 13, dans lequel le dispositif d'immobilisation peut être utilisé pour couvrir une tête et limiter le mouvement du patient par rapport aux poutres.

15. Procédé selon l'une quelconque des revendications 9 à 14, dans lequel le dispositif d'immobilisation est tel que revendiqué selon l'une quelconque des revendications 1 à 8.
